# EUROPEAN PATENT APPLICATION

(11) **EP 4 835 462 A1**
(43) Date of publication of application: **04.11.2026**
(21) Application number: 24909339.4
(22) Date of filing: 19.01.2024
(51) Int. Cl.: G01N 27/416, G01N 27/327, G01N 33/487

(54) **METHOD FOR TESTING ANALYTE IN BIOLOGICAL SAMPLE, SENSOR, AND SYSTEM**

(30) Priority: 28.12.2023 CN 202311851595
(71) Applicant: Jiangsu Yuwell POCTech Biotechnology Co., Ltd., Zhenjiang, Jiangsu 212300 (CN)
(72) Inventor: WEI, Liang, Zhenjiang, Jiangsu 212300 (CN); LI, Chengtao, Zhenjiang, Jiangsu 212300 (CN); WANG, Caiping, Zhenjiang, Jiangsu 212300 (CN); WANG, Hui, Zhenjiang, Jiangsu 212300 (CN); WEI, He, Zhenjiang, Jiangsu 212300 (CN); ZHAO, Shuai, Zhenjiang, Jiangsu 212300 (CN); WU, Qun, Zhenjiang, Jiangsu 212300 (CN)
(74) Representative: Lorenz Seidler Gossel Part. mbB
(86) International application number: PCT/CN2024/073155
(87) International publication number: WO 2025/138367

(57) **Abstract**

A method for testing an analyte in a biological sample, a sensor, and a system. The method includes: contacting a first working electrode (D) and a second working electrode (A) with a biological sample, and inputting a first excitation signal and a second excitation signal to the first working electrode (D); inputting, between the first excitation signal and the second excitation signal, an excitation signal sequence to the second working electrode (A); measuring, on the first working electrode (D), at least one first output signal in response to the first excitation signal, at least one second output signal in response to the second excitation signal, and at least one output signal sequence in response to the excitation signal sequence; and obtaining a concentration of the analyte in the biological sample based on the first output signal, the second output signal, and the output signal sequence. The first output signal, the second output signal, and the output signal sequence are used as parameters for calculating the concentration of the analyte in the biological sample, so that the impact of interfering materials can be eliminated, thereby improving the measurement accuracy of the test method.

## Description

This application claims priority to Chinese Patent Application No. 202311851595.7, filed with the China National Intellectual Property Administration on December 28, 2023 and entitled "METHOD FOR TESTING ANALYTE IN BIOLOGICAL SAMPLE, SENSOR, AND SYSTEM", which is incorporated herein by reference in its entirety.

### TECHNICAL FIELD

This application relates to a method for testing an analyte in a biological sample, a sensor, and a system, and relates to the technical field of biological sample analysis.

### BACKGROUND

When an analyte in a biological sample is measured and analyzed, an input signal is applied to a working electrode of a sensor by a measurement device. The working electrode transfers the input signal to the biological sample, so that the analyte in the biological sample undergoes an oxidation-reduction reaction, to generate an output signal in response to the input signal, so as to determine a concentration of the analyte in the biological sample based on the output signal. The analyte includes materials, such as blood glucose, blood ketones, blood lactic acids, choierols, uric acids, triglycerols, blood clotting factors, and anti-clotting factors, and the test result of the analyte can be used for medical diagnosis and treatment of physiological abnormalities. For example, a diabetic individual may use a biosensor system to measure a glucose level in blood to adjust diets and/or medicines.

In a conventional biosensor, test selectivity is achieved by using a biomolecule catalyzed single reaction and is implemented based on two chain reactions. A reduced state of a mediator of the second reaction (a comb flow reaction) diffuses to an electrode, and is then oxidized to generate a corresponding current signal. A catalytic process on an analyte may be described by using the following three steps:
(1) $analyte + {enzyme}_{oxidized state} = biochemical reaction product + {enzyme}_{reduced state}$
(2) ${mediator}_{oxidized state} + {enzyme}_{reduced state} = {mediator}_{reduced state} + {enzyme}_{oxidized state}$
(3) ${mediator}_{reduced state} \left(electrode\right) = {mediator}_{oxidized state} + {e}^{-}$

Enzyme biomolecules in a glucose measurement sensor system unitarily catalyze oxidation of glucose molecules in blood. However, through the reaction of the added mediator with the enzyme, the enzyme is converted from the reduced state back to the oxidized state, so as to implement a comb flow function. A gluconase may be an oxidase, or may be a dehydrogenase. Due to the fact that an oxidase is subject to oxidation by dissolved oxygen in a blood sample and a dehydrogenase is not subject thereto and therefore does not have an "oxygen dissolving effect", dehydrogenases have been the first choice of enzymes in biosensors in the recent two decades. A catalase of another analyte may be an oxidase, or may be a dehydrogenase, and the principles of the biosensors are substantially the same. However, in a test process, although an enzyme-catalyzed reaction is substantially unitary, other interfering materials capable of being oxidized may exist in a blood sample. These interfering materials may be subject to an oxidization reaction, to generate an interfering signal. Generally, a higher standard potential of a mediator of a comb flow reaction, for example, iron cyanide (Fe(CN)₆⁻³), indicates a higher proneness of generating an interfering signal, because a higher electrode oxidization potential indicates oxidization of more interfering materials.

In addition to the chemical interfering materials, other test interferences may further exist in the biosensors. For example, the percentage of hematocrit (%-HCT) is a relatively significant interfering factor. The interference is caused by that diffusion of the mediator, in a process in which the mediator undergone the comb flow reaction is diffused to the electrode, is hampered by blood cells, so that a diffusion coefficient changes along with the %-HCT. A current in an electro-chemical reaction may be affected by different %-HCTs. Therefore, delivery batch calibration of a sensor test strip is usually centered on the HCT of 42%. If the %-HCT of a user is higher than 42%, a negative deviation occurs in the test. However, if the %-HCT of a user is lower than 42%, a positive deviation occurs in the test. In addition, an aging effect generated during a storage time of a test strip may further interfere with a test result, that is, as a storage time is prolonged, sensitivity of a sensor test relatively declines, or more oxidizable materials available for a background signal may be generated. In addition, the interfering factors further include a test temperature.

In a conventional biosensor, a reduced state mediator generated after a comb flow reaction is oxidized by means of applying a voltage to an electrode in contact with an analyte sample, so as to generate a current signal in response to the analyte. The current signal is converted into a concentration of the analyte by using a corresponding calculation method. For an interfering factor in a test process, in most of existing methods, different single parameters are used to compensate for interference exclusion or reduction, so as to improve the test accuracy and the precision. However, in the single parameter interference compensation methods, mutual impact relationships among a plurality of interfering factors are usually ignored, for example, mutual impact between a temperature and an HCT, mutual impact between a temperature and aging of a test strip, mutual impact between an HCT and aging of a test strip, and the like.

For a long run, conventional biosensors are configured to generate an output signal by means of single excitation, and determine a concentration of an analyte based on the single output signal. For a multi-pulse excitation method developed later, for example, the method disclosed in CN 109690304 A, provides a plurality of parameters to improve the accuracy and the precision of a sensor, so as to overcome impact of chemical interfering materials and provide discrimination between a whole blood sample and a quality control liquid, and desirably improve the performance of a biosensor. However, in the method, pulse signals need to be alternately input to two working electrodes. Test vibration easily occur to affect the continuity of a subsequent input signal, resulting in an incorrect signal that is difficult to be identified, and consequently, the accuracy of a test result is reduced due to excessive uncertainty factors.

Therefore, to avoid generation of an interfering signal, the method for testing an analyte in a biological sample can be improved to a relatively large extent in aspects such as multi-pulse excitation and multi-parameter compensation, so as to further improve the accuracy and the precision of testing the analyte in the biological sample.

### SUMMARY

To resolve the foregoing problem, the inventors propose an apparatus, a system, and a method for testing an analyte in a biological sample. The proposed apparatus, system, and method are correlated with a concentration of one or more analytes in a tested biological sample. According to the method, at least two excitation signals are input to a first working electrode, and an excitation signal sequence is input to a second working electrode between the at least two excitation signals for the first working electrode, the input signals for the first and second working electrodes do not overlap in time, that is, after the excitation signal for the first working electrode is completed, the first working electrode is controlled by the system to be in an open circuit state, and then input of an excitation signal sequence to the second working electrode is started; and after the excitation signal sequence for the second working electrode is completed, the second working electrode is controlled by the system to be in an open circuit state, and then another excitation signal for the first working electrode is started. The excitation signal input by the first and the second working electrode is defined as a "sandwiched pulsing excitation method" (sandwiched pulsing excitation). The first working electrode is covered by a first chemical agent capable of chemically reacting with a predetermined analyte, and the first chemical agent includes a biological enzyme capable of catalyzing reaction of an analyte, one or more mediators (mediator) capable of generating a comb flow function between an enzyme reaction and an electrode reaction, and an inactive material assisting the chemical agent. A chemical agent covered on the first counter electrode corresponding to the first working electrode includes at least a mediator, or has a composition the same as that of the first chemical agent. The second working electrode is a blank electrode or is covered with an inactive material that does not chemically react with the analyte. Corresponding to the foregoing sandwiched pulsing excitation method, the sensor system measures output signals generated by input excitation signals, that is, output signals generated from the first and second excitation signals input to the first electrode and an output signal series generated by the excitation signal series input to the second electrode. Meanwhile, the measured signal further includes a remaining attenuated signal generated by a corresponding electrode in a conjugate reaction with the first electrode and generated by the first electrode after the first excitation signal is stopped, a remaining attenuated signal that is generated by the first electrode and that is generated after the second excitation signal of the first electrode is stopped, and the like.

An aspect of the disclosure of the sandwiched pulsing excitation method is used for determining one or more analytes in a biological sample, and determining a concentration of an analyte from one or more signal values in the first and second output signals of the first electrode, one or more signal values in the output signal series of the second electrode, and remaining attenuated signal values of the first and second input signals to the first electrode.

Another aspect of the disclosure of the sandwiched pulsing excitation method, especially, an output signal series value generated by using a sandwiched input excitation signal series, is used for determining other parameters in the biological sample, including a hematocrit value (%-HCT) of a whole blood sample, and providing proper compensation and correction to measurement of the analyte by using a parameter correlated with the hematocrit value, to exclude or reduce impact of blood cells on the measurement of the analyte, so as to improve the accuracy and the precision of the measurement of the analyte.

### BRIEF DESCRIPTION OF THE DRAWINGS

FIG. 1 is a schematic diagram of a sensor for testing an analyte in a biological sample according to the present invention.
FIG. 2 is a schematic diagram of a main excitation signal in a sandwiched pulsing excitation method according to this embodiment.
FIG. 3 is a schematic diagram of different step waves in a sandwiched pulsing excitation method.
FIG. 4 illustrates output current signals of different step waves at different blood glucose concentrations.
FIG. 5A is a schematic diagram of another implementation manner of a multi-pulse sandwiched pulsing excitation method.
FIG. 5B is a schematic diagram of still another implementation manner of a multi-pulse sandwiched pulsing excitation method.
FIG. 5C is a schematic diagram of yet another implementation manner of a multi-pulse sandwiched pulsing excitation method.
FIG. 6A is a diagram of output currents of a sandwiched pulsing excitation method at different blood glucose concentrations.
FIG. 6B is a linear relationship diagram of an output current for an excitation signal of a first working electrode and a blood glucose concentration in a sandwiched pulsing excitation method.
FIG. 6C is a diagram of output currents of a multi-pulse sandwiched pulsing excitation method at different blood glucose concentrations.
FIG. 6D is a linear relationship diagram of an output current for an excitation signal of a first working electrode and a blood glucose concentration in a multi-pulse sandwiched pulsing excitation method.
FIG. 6E is a diagram of output currents of different step waves at different blood glucose concentrations.
FIG. 7A is a potential attenuation curve of a first excitation wave applied to a first working electrode at different blood glucose concentrations after a first excitation wave applied to the first working electrode ends.
FIG. 7B is a diagram showing a relationship between a relative attenuation coefficient of an attenuation potential at a first working electrode and an analyte reference concentration.
FIG. 7C is a potential attenuation curve of a second excitation wave applied to a first working electrode at different blood glucose concentrations after a first excitation wave applied to the first working electrode ends.
FIG. 7D is a diagram showing a relationship between an attenuation potential at a first corresponding electrode and a concentration of an analyte.
FIG. 8 is a diagram of output currents corresponding to step waves of a second working electrode at different red cell packed volumes.
FIG. 9A is a linear relationship diagram of a current of a fourth step wave and a hematocrit at different blood glucose concentrations.
FIG. 9B is a diagram of a ratio of a current value of the fourth step wave at different hematocrits to a current value of the fourth step wave at a hematocrit of 42%.
FIG. 9C is a linear relationship diagram of a current of a fifth step wave and a hematocrit at different blood glucose concentrations.
FIG. 9D is a diagram showing a ratio of a current value of a fifth step wave at different hematocrits to a current value of the fifth step wave at a hematocrit of 42%.
FIG. 10A a linear relationship diagram of Δi₄₃ and a hematocrit at different blood glucose concentrations.
FIG. 10B is a diagram showing a relationship between a ratio of a current value Δi₄₃ at different hematocrits to a current value Δi₄₃ at a hematocrit of 42% and a hematocrit.
FIG. 10C is a linear relationship diagram of Δi₅₄ at different blood glucose concentrations and a hematocrit.
FIG. 10D is a diagram showing a relationship between a ratio of a current value Δi₅₄ at different hematocrits to a current value Δi₅₄ at a hematocrit of 42% and a hematocrit.
FIG. 10E is a linear relationship diagram of Δi₅₃ at different blood glucose concentrations and a hematocrit.
FIG. 10F is a diagram showing a relationship between a ratio of a current value Δi₅₃ at different hematocrits to a current value Δi₅₃ at a hematocrit of 42% and a hematocrit.
FIG. 11 illustrates an exemplary embodiment of a linear relationship diagram between ii_{average} and a glucose concentration of the present invention.
FIG. 12 is a comparison diagram of deviations before and after calibration in Embodiment 1.
FIG. 13 is a comparison diagram of deviations before and after calibration in Embodiment 2.
FIG. 14 is a comparison diagram of deviations before and after calibration in Embodiment 3.
FIG. 15 is a comparison diagram of deviations before and after calibration in Embodiment 1 to Embodiment 3.
FIG. 16 is a schematic diagram of a measurement circuit according to an embodiment of this application.

### DETAILED DESCRIPTION

The excitation described in the disclosure of the present invention means applying an input signal to an electrode in a sensor, where the applied signal includes constant positive and negative direct-current voltage sources, or constant positive and negative direct-current sources, or the like, so that a complete or partial reaction on the surface of the electrode is carried out on the surface of the electrode. A system processing center of the sensor applies an excitation input signal to a corresponding electrode via a connection port between an instrument and a sensor test strip. After a predetermined excitation time ends, the system no longer applies a signal to the electrode, and the electrode is in an open circuit state. The open circuit state does not mean a zero input. For example, an input of a voltage of 0 V (zero volt) to an electrode is not equivalent to an open circuit. A zero voltage is also an input voltage. After the electrode enters the open circuit state, the material in an oxidized-reduced state on and near the surface of the electrode may cause an attenuation process of a remaining signal. The attenuated signal may reflect a state that the electrode has ever been in, so as to record other information of the sensor correlated with the analyte.

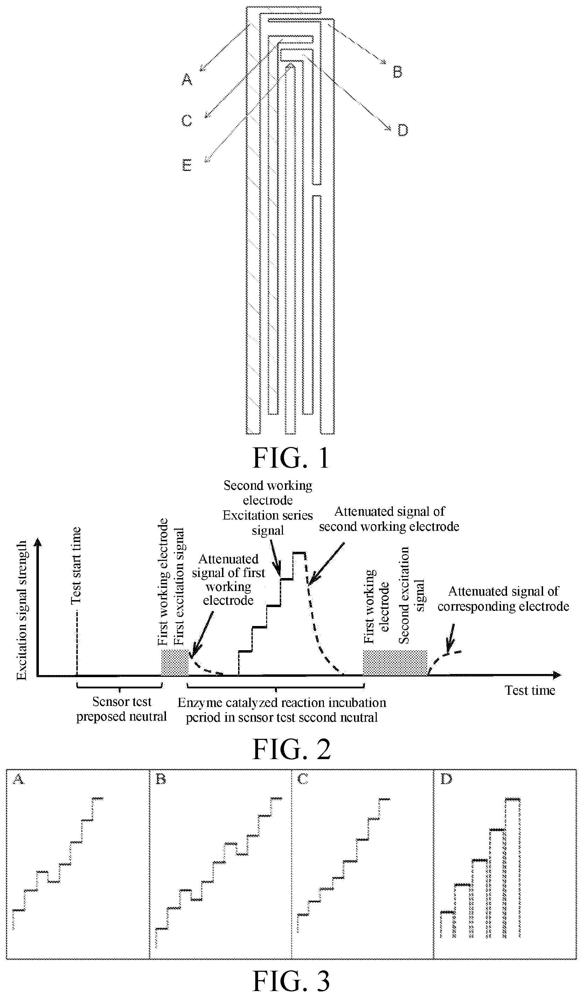

FIG. 1 is a schematic diagram of a sensor. D represents a first working electrode, and is covered with a first chemical agent corresponding to a predetermined analyte. C represents a first counter electrode corresponding to the first working electrode and is covered with at least a particular amount of a mediator or a chemical agent the same as that of the first working electrode. E represents a sample feeding measurement electrode. A represents a second working electrode in a sensor relative to a sandwiched pulsing excitation method. The second working electrode may be one of an electrode combination configured to measure an impedance in the sensor. A surface of the second working electrode may be blank, or may be covered with an inactive material or a second chemical agent different from the first chemical agent. B is a second counter electrode corresponding to the second working electrode.

The first chemical agent includes an oxidation enzyme and a mediator. The oxidation enzyme is selected from at least one of a FAD dehydrogenase; and a PQQ dehydrogenase. The mediator is selected from one of hexaammineruthenium (III) chloride, potassium ferricyanide, a ferrocene derivative, phenazine methosulphate, and a polyethersulfone resin. The inactive material is selected from at least one of carboxymethyl cellulose, hydroxymethyl cellulose, polyethylene glycol, and polyvinyl pyrrolidone.

One of the related disclosure of the present invention is a sandwiched pulsing excitation method. FIG. 2 is a schematic diagram of main elements of a sandwiched pulsing excitation method. First, a first excitation signal is applied to the first working electrode. After the first working electrode is turned off, an excitation signal sequence (that is, the excitation series signals in the figure) is continuously applied to the second working electrode. After the second working electrode is turned off, a second excitation signal is again applied to the first working electrode.

FIG. 2 is a schematic diagram of a main excitation signal in a sandwiched pulsing excitation method according to this embodiment. An x-axis is a test time of a sensor. A y-axis represents an excitation signal strength, and may be a voltage input to an electrode, may be a constant current input to the electrode, or the like. There is a front test neutral in the figure, which is referred to as a first neutral. In the neutral, the sensor does not accept any input signal. A time range of the first neutral ranges from 0s to 2s, where 0s represents that the sensor system immediately starts a test program after determining that the sample completely fills an empty cavity of the sensor, and the time range of the neutral between 0s to 2s provides enzyme catalyzed reaction and incubation to the first working electrode of the sensor before the first excitation signal is applied thereon. In addition, the first neutral may alternately be used for measuring an impedance of a sample by using a particular electrode pair.

After the first neutral, a first excitation signal is applied to the first working electrode. An excitation voltage of the first excitation signal is 0.2 V to 0.4 V, preferably 0.3 V, and an excitation time is 0.1s to 1s, preferably 0.5s.

After the first excitation signal is completed, the system turns off the first working electrode. This is a second neutral of the first working electrode. During the second neutral, an active area of the first working electrode performs an enzyme catalyzed reaction, to incubate and accumulate a reduced state mediator after a comb flow reaction, to prepare for an electro-oxidation reaction of the second excitation signal. Meanwhile, the sensor system may perform other tests. For example, an attenuated signal generated after the first excitation signal is applied on the first working electrode is measured, and a measurement time is between 0.005s and 0.5s.

Then, the system starts an excitation signal sequence for the second working electrode. Time of each excitation in the excitation signal sequence may range from 0.05s to 0.5s, may be a successive excitation sequence with no intervals, or may be a combination of different excitation pulses with an interval. Preferably, the excitation signal sequence is a successive excitation sequence with no intervals. It may be understood that, the successive excitation sequence may be a successively increasing excitation sequence, or may be an excitation sequence that increases and then decreases, or an excitation sequence that increases and then decreases and then increases, or the like. Preferably, the successive excitation sequence is a successively increasing excitation sequence. If the excitation series signal is a voltage, a voltage value is between 0.1 V and 3 V. The excitation series signal shown in the figure includes five excitations, excitation voltages of the five excitations may be sequentially 0.3 V, 0.8 V, 1.2 V, 1.7 V, and 2.2 V, and a time of each excitation may be 0.2s. This excitation series signal may be referred to as a "step wave". After the excitation signal sequence of the second working electrode ends, the system sets the second working electrode to an open circuit state, and measures an attenuated signal of the second working electrode after the second working electrode is turned off. A measurement time is between 0.005s and 0.5s.

Finally, the system applies a second excitation signal to the first working electrode. An excitation time is between 0.1s and 2s, preferably 1.25s, and an excitation voltage is between 0.2 V and 0.4 V, preferably 0.3 V. After the second excitation signal ends, the system sets the first working electrode to an open circuit state, and measures a potential attenuated signal of a corresponding electrode in the system. A measurement is between 0.005 and 0.5.

In combination with the foregoing descriptions, preferably, a test time and a potential in the test method are controlled as follows: (1) preposing a test neutral of 1.25s; (2) applying a first excitation signal to the first working electrode, inputting a potential difference of 0.3 V to the first working electrode and a corresponding electrode, lasting for 0.5s, and synchronously measuring output current signals at an interval of 0.05s; (3) after the first excitation signal ends, setting the first working electrode to an open circuit state, and measuring a first potential attenuated signal of the first working electrode at an interval of 0.005s, lasting for 0.5s; (4) when the first working electrode is continuously in an open circuit state, inputting an excitation signal sequence to the second working electrode and the corresponding electrode, the excitation signal sequence including five excitations, excitation voltages thereof sequentially being 0.3 V, 0.8 V, 1.2 V, 1.7 V, and 2.2 V, duration of each excitation being 0.2s, and synchronously measuring output current signals (a step wave 1 for short below) at an interval of 0.05s; (5) setting the second working electrode to an open circuit state; (6) after the first working electrode is continuously in an open circuit state for 2.0s, applying a second excitation signal to the first working electrode at a voltage of 0.3 V (volt) for 1.25s, and synchronously measuring output current signals at an interval of 0.05s; and (7) after the second excitation signal ends, setting the first working electrode to an open circuit state, and measuring a potential attenuated signal of the corresponding electrode at an interval of 0.005s, lasting for 0.5s. A total time of the foregoing test steps is 1.25s+0.5s+2s+1.25s+0.5s=5.5s.

In addition to the excitation signal sequence shown in FIG. 2 and implemented for the second working electrode, other different excitation series signals, that is, step waves, may alternately be implemented. FIG. 3 illustrates several different cases of step waves in a sandwiched pulsing excitation method: (1) a second step wave shown in FIG. 3A: after excitation voltages of excitation signals successively increase by three steps, the excitation voltages of the excitation signals fall back by one step and then successively increase by four steps, the voltages of the excitation signals are sequentially 0.3 V, 0.7 V, 1.1 V, 0.9 V, 1.2 V, 1.5 V, 1.9 V, and 2.3 V, and the voltage of each excitation signal lasts for 0.15s; (2) a third step wave shown in FIG. 3B: after excitation voltages of excitation signals successively increase by three steps, the excitation voltages of the excitation signals fall back by one step, then successively increase by three steps, then fall back by one step, and successively increase by another three steps, the voltages of the excitation signals are sequentially 0.3 V, 0.6 V, 0.9 V, 0.8 V, 1.1 V, 1.4 V, 1.7 V, 1.6 V, 1.9 V, 2.3 V, and 2.7 V, and the voltage of each excitation signal lasts for 0.15s; (3) a fourth step wave shown in FIG. 3C: increase amplitudes of first several excitation signals and last several excitation signals in an excitation signal sequence are different, and voltages of the excitation signals are sequentially 0.3 V, 0.5 V, 0.7 V, 0.9 V, 1.2 V, 1.6 V, 2.0 V, and 2.4 V, and each voltage lasts for 0.15s; (4) a fifth step wave shown in FIG. 3D: an excitation signal sequence is a non-successive excitation signal, each excitation pulse signal is separated by a short open circuit time, and an interval time does not exceed 50% of a pulse excitation time. The voltages of the excitation signals are sequentially 0.3 V, 0.8 V, 1.2 V, 1.7 V, and 2.2 V. Each excitation pulse lasts for 0.15s. There is an open circuit interval of 0.05s between the pulses.

According to solutions 2, 3, and 4 of the excitation signal sequence, an output signal with relatively low excitation strength and an output signal with relatively high excitation strength may be acquired. For example, when an input signal is a potential, a relatively common chemical in a sample is more suitable for measurement between 0.3 V and 1.2 V, and when the input signal is between 1.2 V and 3.0 V, a chemical that needs high-intensity excitation is excited, such as a hematocrit value. Finally, relatively other output signals may be generated from the non-successive interval pulse excitation series signals.

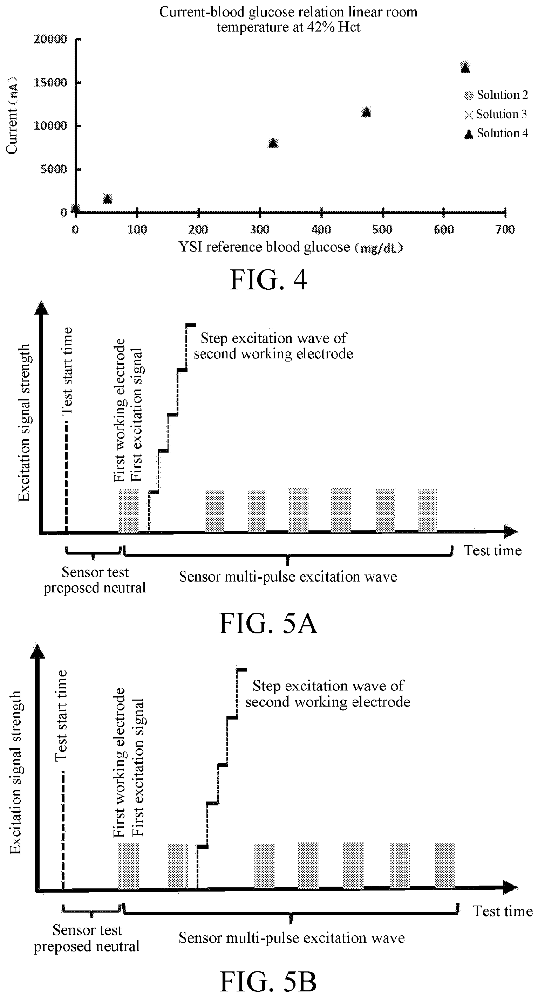

FIG. 4 illustrates output current signals of the second excitation signal at different blood glucose concentrations when the step wave between the first excitation signal and the second excitation signal is solution 2, 3, or 4. As can be seen from the figure, an excitation magnitude and type of the step wave have no direct impact on the output current signal of the second excitation signal.

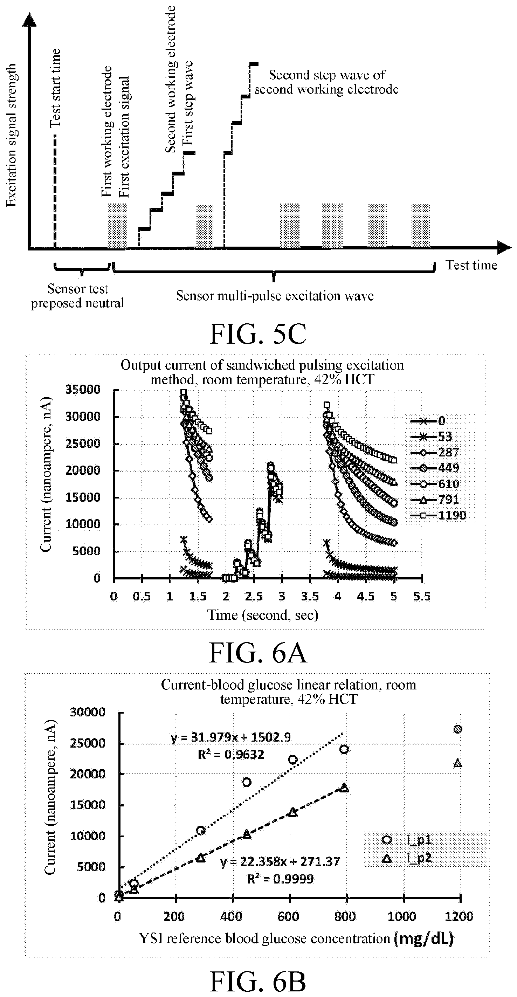

As shown in FIG. 4, in continuation of the working method in FIG. 2, there may be two or more excitation signals applied to the first working electrode, and the step wave of the second working electrode is sandwiched between two or three excitation waves. FIG. 5A, FIG. 5B, and FIG. 5C illustrate other multiexcitation sandwiched pulsing excitation methods. In FIG. 5A, the excitation signal sequence (a step wave) of the second working electrode is sandwiched between first and second excitation waves of the first working electrode. The step wave signal may be a step wave shown in FIG. 3. Subsequently, subsequent multi-pulse excitation is performed on the first working electrode. Each pulse of the excitation wave applied to the first working electrode is constant-value excitation. Application time of each excitation may be the same or may be different, and duration of each excitation being between 0.2s and 2s. The open-circuit time between each excitation wave may be equal or different, and the open-circuit time is between 0.2s and 5s. In FIG. 5B, the excitation signal sequence of the second working electrode is sandwiched between the second and third excitation waves of the first working electrode. The excitation signal sequence may be a step wave case in FIG. 3. Then, a subsequent multi-pulse excitation wave is applied to the first working electrode. An excitation wave applied to the first working electrode is constant-value excitation, and an application time of each excitation may be the same or may be different. An excitation duration is between 0.2s and 2s. The open-circuit time between each excitation wave may be equal or different, and the open-circuit time is between 0.2s and 5s. In FIG. 5C, two excitation signal sequences (step waves) are applied to the second working electrode. The first step wave is sandwiched between the first and second excitation waves of the first working electrode, and the second step wave is sandwiched between the second and third excitation waves. Then, a subsequent multi-pulse excitation wave is applied to the first working electrode. The first step wave of the second working electrode is a low-intensity excitation. For example, an excitation potential may be 0.15 V to 1.2 V. The second step wave is a high-intensity excitation. For example, an excitation potential may be 1.2 V to 2. 5 V. An excitation wave applied to the first working electrode is constant-value excitation, and an application time of each excitation may be the same or may be different. An excitation duration is between 0.2s and 2s. The open-circuit time between each excitation wave may be equal or different, and the open-circuit time is between 0.2s and 5s.

FIG. 6A illustrates an output current when two excitation signals and one step wave are included in the sandwiched pulsing excitation method. 0, 53, 287, ..., and 1190 in the figure are respectively blood glucose reference concentrations (mg/dL). It can be seen that the output current of the step wave is irrelevant to the blood glucose concentration. FIG. 6B is a linear relationship diagram between the output current of the excitation signal of the first working electrode and the blood glucose concentration in the sandwiched pulsing excitation method. i_p1 is an output current of the first excitation wave and i_p2 is an output current of the second excitation wave. Excitation and current acquisition of the first excitation wave are completed within 1.75s. In this case, the sensor chemical is still in a meta-stable state, the current is quite high, but the background current is also quite high, so that the ratio of the intercept to the slope of the response regression line is at approximately 47 mg/dL. The second excitation wave is started at 3.75s and completed at 5s, and a current is acquired. The slope is slightly lower than that of the response line of the first excitation wave, but the background current greatly decreases, so that the ratio of the intercept to the slope is approximately 12.1 mg/dL. After the blood glucose concentration reaches 1190 mg/dL, a linear relationship formed by the current output by the first excitation wave and the current output by the second excitation wave is slightly lower than a linear relationship formed at a low concentration (0-800 mg/dL). It is proved that the method has a relatively wide measurement range.

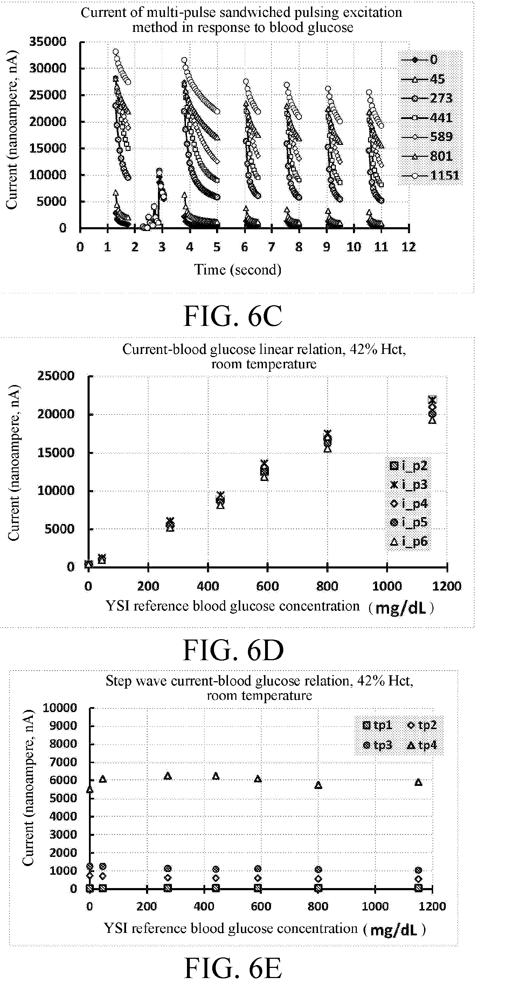

FIG. 6C is output currents of six pulse excitations performed on the first working electrode in the sandwiched pulsing excitation method. In the figure, 0, 45, 273, ..., 1151 are respectively blood glucose reference concentrations (mg/dL). A measurement upper limit of the blood glucose concentration may be approximately 1200 mg/dL, which is almost twice of the upper limit of a blood glucose meter (600 mg/dL) commonly used in the market. There is an incubation period before each excitation wave, that is, a first neutral and a second neutral and subsequent open-circuit and closed-circuit multi-pulse excitations in FIG. 2. Compared with an existing test method, that is, a voltage is immediately input to an electrode upon starting of a test, there is a neutral before each excitation signal in the present test method. Accumulation of an oxidizable mediator brought in the neutral as an incubation period ensures that a current generated by a first excitation wave is quite stable, and may be used as one of parameters for calculating a concentration of an analyte. A second neutral lasts a longer time, and more enzyme reaction product is interacted and accumulated, so that a current generated by the second excitation wave is more stable, and the repeatability is better. A linear relationship between an output current of another multi-pulse excitation wave and a blood glucose concentration can be seen from FIG. 6D. A linear relationship between output currents (i_p2, i_p3, i_p4, i_p5, i_p6) from the second excitation wave to the sixth excitation wave changes little. As can be seen from FIG. 6D, when the blood glucose concentration is 0 mg/dL to 800 mg/dL, the output currents from the second excitation wave to the sixth excitation wave have a good linear relationship with the blood glucose concentration, and when the blood glucose concentration is 800 mg/dL to 1150 mg/dL, the linear relationship is slightly poor, but there is still a linear relationship. Finally, FIG. 6E illustrates an output current of a step wave, which almost keeps unchanged at different blood glucose concentrations. This feature exactly forms an independent parameter irrelevant to the output current of the first working electrode, for testing other blood glucose-related performance parameters. tp1 represents an output current of the first step wave, tp2 represents an output current of the second step wave, tp3 represents an output current of the third step wave, and tp4 represents an output current of the fourth step wave.

Table 1 summarizes results of the output currents of six pulsing excitation waves, including slopes, intercepts, and linear correlation coefficients. Except for the first excitation wave, the linear correlation coefficients of other excitation waves are all greater than 0.99. Linear constants of the second to the fourth excitation waves are quite close. Linear constants of the fifth and sixth excitation waves descend, but are also quite close. Proximity of the second to the sixth excitation waves may be seen from the slope, the intercept, the correlation coefficient, and the intercept/slope ratio, respectively. A multi-pulse output current provides more parameters correlated with the blood glucose, so that the calculation accuracy of a test result can be improved.

**Table 1**

| **Items** | **Endpoint time (seconds)** | **Excitation wave** | **Slope** | **Intercept** | **Correlation coefficient, R²** | **Slope/Intercept** |
|---|---|---|---|---|---|---|
| **First excitation wave** | 1.75 | i_p1 | 27.7 | 1412.6 | 0.9816 | 51 |
| **Second excitation wave** | 5 | i_p2 | 20.7 | 255.0 | 0.9987 | 12.3 |
| **Third excitation wave** | 6.5 | i_p3 | 21.7 | 289.8 | 0.9979 | 13.4 |
| **Fourth excitation wave** | 8 | i_p4 | 20.9 | 227.3 | 0.9974 | 10.9 |
| **Fifth excitation wave** | 9.5 | i_p5 | 20.1 | 179.2 | 0.9974 | 8.9 |
| **Sixth excitation wave** | 11 | i_p6 | 192 | 139.8 | 0.9973 | 7.3 |

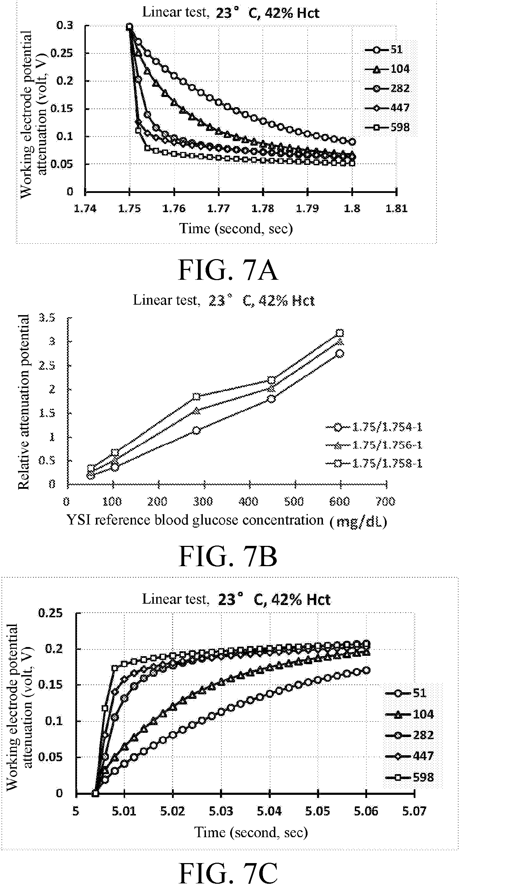

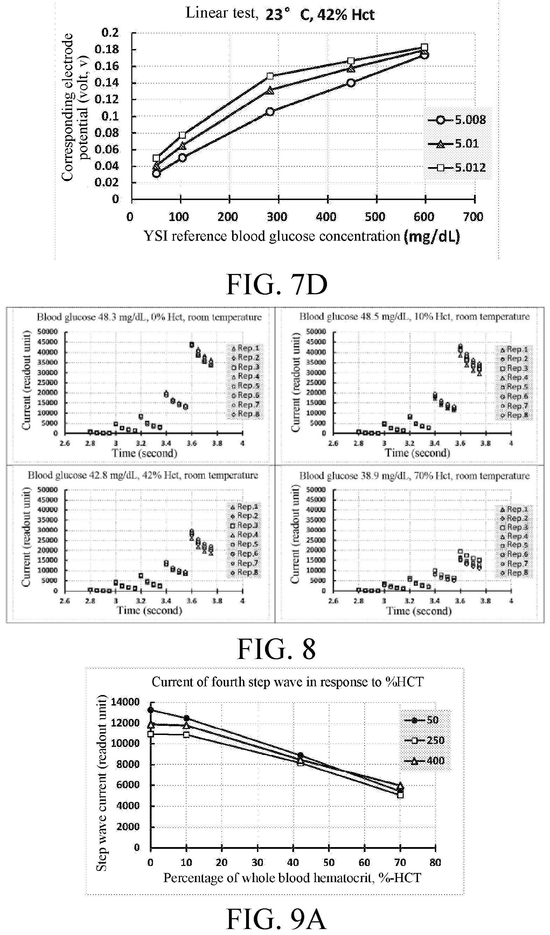

FIG. 7A illustrates a potential attenuation curve generated by the first working electrode for different concentrations of the analyte after the first excitation wave applied to the first working electrode ends (that is, an open circuit). 51, 104, 282, 447, and 598 in the figure are respectively blood glucose reference concentrations (mg/dL). The group of attenuation curves indicates that, after excitation of the first working electrode by the first excitation wave ends, a potential attenuation speed of the first working electrode is in direct proportion to the concentration of the analyte, that is, a higher concentration of the analyte indicates a faster attenuation speed, so that within a particular time shortly after the first working electrode is turned off, a higher concentration of the analyte indicates a lower output attenuation potential. The attenuation speed may be expressed by using a relative attenuation coefficient, and may be obtained through the following calculation: that is, a potential at a time point obtained after attenuation is subtracted from an initial potential (approximately 0.3 V), and then divided by the potential at the time point. FIG. 7B is a diagram showing a relationship between a relative attenuation coefficient of an attenuation potential at a first working electrode and an analyte reference concentration. In the figure, 1.75/1.754-1=V1.75s/V1.754s-1. Similarly, 1.75/1.756-1=V1.75s/V1.756s-1, and 1.75/1.758-1=V1.75s/V1.758s-1. The figure indicates that, after excitation of the first working electrode ends (an open circuit), the relative attenuation coefficient may be used to display the concentration of the analyte. The three relative attenuation coefficients are in a near-linear relationship with the blood glucose concentration. FIG. 7C is a potential attenuation curve of the first corresponding electrode after applying of the second excitation wave to the first working electrode (open circuit) by the sensor system ends. 51, 104, 282, 447, and 598 in the figure are respectively blood glucose reference concentrations (mg/dL). A potential difference relationship between the first working electrode and the first corresponding electrode is that the first corresponding electrode is set to zero, that is, an instrument ground line, and a corresponding excitation potential is input to the first working electrode. Therefore, attenuation at the first corresponding electrode starts to increase from zero and belongs to a negative attenuation relationship. FIG. 7D is a diagram showing a relationship between an attenuation potential at a first corresponding electrode and a concentration of an analyte. 5.008 in the figure indicates an attenuation potential at the 5.008^{th} second, 5.01 indicates an attenuation potential at the 5.01^{th} second, and 5.012 indicates an attenuation potential at the 5.012^{th} second. The figure indicates that a potential attenuation quantity of the first corresponding electrode is in an almost linear direct proportional relationship with the concentration of the analyte. It can be seen from the foregoing potential attenuations of the first working electrode and the first corresponding electrode that, an attenuation potential may be used as an auxiliary parameter of the concentration of the analyte, thereby increasing the accuracy and the precision for measuring the concentration of the analyte. For example, the concentration of the analyte may be expressed by using a multi-parameter regression equation: G=f(ip1, ip2, v1, 1, v1, 2, v1, 3, v2, 1, v2, 2, v2, 3...).

FIG. 8 illustrates output signals of a second working electrode in five step excitation waves in an embodiment of the step wave. Due to the fact that the second working electrode is not covered by a chemical agent capable of reacting with an analyte, at a predetermined oxidation potential of 0.3 V, a generated current is almost close to zero, that is, the second working electrode does not generate a response current correlated with the blood glucose concentration. As the excitation potential increases, the current generated by the second working electrode also increases. Particularly, when the excitation potential is increased to 1.7 V, the current generated by the second working electrode is quite high, and reaches the highest when the excitation potential is 2.2 V. However, as a hematocrit value (%-HCT) of a whole blood sample increases, a generated current decreases. This is because at a high potential, an oxidized material (glucose cannot be directly oxidized) in blood plasma of the blood is totally oxidized to obtain a current. The HCT represents a quantity of red blood cells. A higher HCT indicates a fewer oxidized material and indicates a lower current. It is intuitively proved that when the second working electrode has a relatively high excitation potential, a current generated by the second working electrode may be used to represent an HCT value.

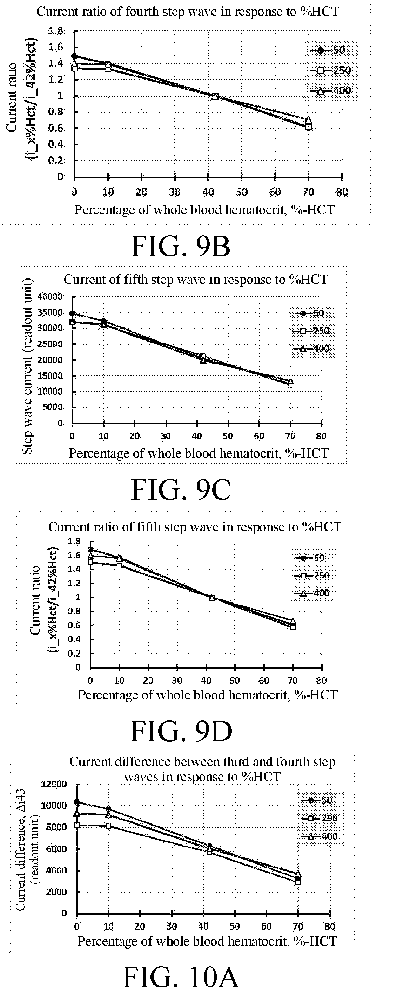

FIG. 9 is a relationship diagram between a related current parameter set extracted from a current generated by the second working electrode and the hematocrit value (%-HCT) in a whole blood sample. 50, 250, and 400 in the figure respectively represent blood glucose concentrations of 50 mg/dL, 250 mg/dL, and 400 mg/dL. FIG. 9A is a linear relationship diagram of a current of a fourth step wave and a hematocrit at different blood glucose concentrations. A y-coordinate of FIG. 9B is a ratio of a current value of the fourth step wave for different hematocrids to a current value of the fourth step wave at a hematocrit of 42%, and an x-coordinate is the hematocrit. FIG. 9C is a linear relationship diagram of a current of a fifth step wave and a hematocrit at different blood glucose concentrations. A y-coordinate of FIG. 9D is a ratio of a current value of a fifth step wave at different hematocrids to a current of the fifth step wave at a hematocrit of 42%, and an x-coordinate is the hematocrit. As can be seen from FIG. 9B and FIG. 9D, the output current of the second working electrode has a significant gradient in response to the HCT. A higher excitation potential (2.2 V relative to 1.7 V) indicates a more significant gradient, and a current is more independent of a concentration of an analyte (blood glucose concentration) of a blood sample.

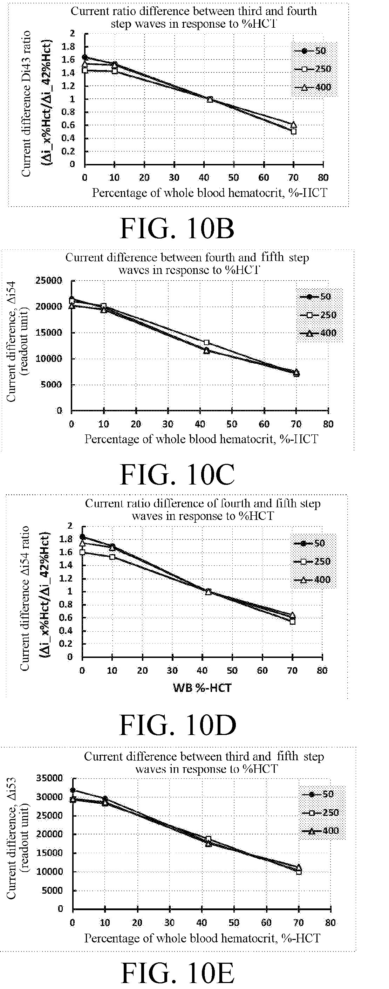

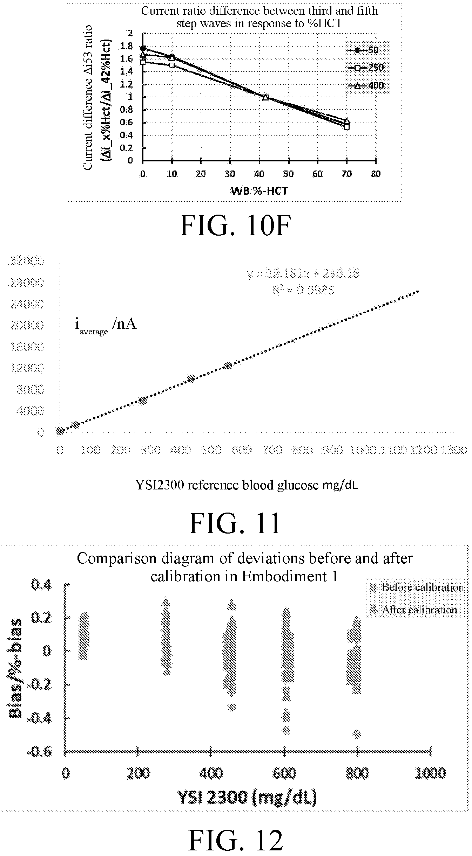

FIG. 10 is a relationship diagram between a related current difference parameter set extracted from a current generated by the second working electrode and the hematocrit value (%-HCT) in a whole blood sample. 50, 250, and 400 in the figure respectively represent blood glucose concentrations of 50 mg/dL, 250 mg/dL, and 400 mg/dL. A current difference between the third and fourth step waves is Δi43=i4-i3, a current difference between the fourth and fifth step wave is Δi54=i5-i4, and a current difference between the third and fifth step wave is Δi53=i5-i3. FIG. 10A is a linear relationship diagram of Δi43 and hematocits at different blood glucose concentrations. The y-coordinate of FIG. 10B is a ratio of a current value of Δi43 at different hematocrits to Δi43 at the hematocrit of 42%, and the x-coordinate thereof is the hematocrit. FIG. 10C is a linear relationship diagram of Δi54 and the hematocrit at different blood glucose concentrations. The y-coordinate of FIG. 10D is a ratio of a current value of Δi54 at different hematocrits to that of Δi54 at the hematocrit of 42%, and the x-coordinate is the hematocrit. FIG. 10E is a linear relationship diagram of Δi53 and hematocrits at different blood glucose concentrations. The y-coordinate of FIG. 10F is a ratio of a current value of Δi53 at different hematocrits to a value of Δi53 at the hematocrit of 42%, and the x-coordinate is the hematocrit. Compared with the gradients in FIG. 9B and FIG. 9D, the gradients in FIG. 10B, FIG. 10D, and FIG. 10F are increased by 10% to 15%, and are more independent of the impact of the concentration of the analyte. Therefore, in an actual application, the use of a direct current difference as a parameter for calculating an HCT is more reliable, and has a higher resolution. This is why using an excitation series signal is more useful and reliable than using a current generated by single high-potential excitation, and this is one of motivations of the present invention. In addition, for the %HCT of the whole blood sample, an optimal regression equation may alternately be found by using a plurality of parameters, that is, a current difference of more than two currents rather than a single high-potential current, that is, %HCT=f(Δi43, Δi54, Δi53).

### Embodiment 1

The blood glucose concentration calculation method is applied to a biological sample analyte test apparatus. The biological sample analyte test apparatus includes a first working electrode and a second working electrode. The first working electrode is covered with a first chemical agent capable of reacting with the analyte. Specifically, the first chemical agent includes a biological enzyme capable of catalyzing reaction of an analyte, one or more mediators capable of generating a comb flow function between an enzyme reaction and an electrode reaction, and an inactive material assisting the chemical agent. The second working electrode is a blank electrode and is covered with an inactive material that does not chemically react with the analyte or covered with a second chemical agent that is different from the first chemical agent, and a first excitation signal and a second excitation signal are input to the first working electrode, where after each excitation signal is completed, the first working electrode is in an open circuit state.

Between the first excitation signal and the second excitation signal, an excitation signal sequence is input to the second working electrode, where after the excitation signal sequence is completed, the second working electrode is in an open circuit state. The excitation signal sequence includes at least two successive or non-successive input signals, each input signal being a constant value;
the first excitation signal, the second excitation signal, and an output current at a current acquisition point of the excitation signal sequence are acquired every given period of time, to obtain an output current group, and obtain a blood glucose concentration. A method for calculating the blood glucose concentration G is obtained by using a formula 1;
formula 1 is $G = \frac{{G}_{calculate}}{1 + f}$. G in the formula 1 is calculated by using a formula 2, f in the formula 1 is calculated by using a formula 3, and f is a multi-parameter calibration factor.

The formula 2 is ${G}_{calculate} = \frac{{i}_{average}}{S}$. i_{average} is an average value nA of output currents i of at least two current acquisition points in the second excitation signal. S is a slope of an i_{average} and glucose concentration linear equation. For example, S is obtained by performing linear fitting based on a known historical i_{average} and a historical glucose concentration. Alternatively, a table is directly listed after the linear relationship between the historical i_{average} and the historical glucose concentration is obtained by using a statistical rule, and the slope S value is obtained by looking up the table.

Formula 3 is $f = K + {\sum }_{n = 1}^{N} \frac{{X}_{n} {i}_{n}}{{i}_{average}} + {i}_{difference} ⋅ {\sum }_{j = 1}^{J} \frac{{X}_{j} {i}_{j}}{{i}_{average}} .$ in and ij are both output currents nA in the output current group; an i_{difference} is a difference nA between a maximum output current of the excitation signal sequence and an output current of the last acquisition point; a value of N ranges from 10 to 53; a value of J ranges from 10 to 53; a value of Xn ranges from -500 to 500; a value of Xj ranges from -15 to 15; and a value of K ranges from -30 to 0.

Optionally, m and u may be output currents of successive acquisition points in the output current group. For example, in may be i1, i2, i3, i4, i5, i6, i7, i8, i9, or i10; ij may be i6, i7, i8, i9, i10, i11, i12, i13, i14, or i15. Optionally, in and ij may be output currents of non-successive acquisition points in the output current group. For example, in may be i3, i5, i11, i13, or i21; ij may be i1, i2, i9, i17, i20, i29, or i30. However, the present invention is not limited thereto, and a person skilled in the art may select values of in and ij based on a statistical significance test method.

Optionally, an excitation time of the first excitation signal is 0.1s to 1s, and an excitation time of the second excitation signal is 0.1s to 2s. Preferably, an excitation time of the first excitation signal is 0.5s, and an excitation time of the second excitation signal is 1.25s.

Optionally, i_{average} is an average value of output currents i of at least three current acquisition points in the second excitation signal. For example, i_{average} is an average value of output currents of last five current acquisition points in the second excitation signal.

Optionally, a voltage value of the first excitation signal is 0.2 V to 0.4 V, and a voltage value of the second excitation signal is 0.2 V to 0.4 V. Preferably, voltage values of the first excitation signal and the second excitation signal are both 0.3 V.

Optionally, a total application time of the excitation signal sequence is at least 1s, and an application time of each input signal in the excitation signal sequence is 0.1s to 0.3s. Preferably, an application time of each input signal in the excitation signal sequence is equal, and is 0.2s.

Optionally, the voltage of the excitation sequence ranges from 0.1 V to 3 V, and the excitation signal sequence includes four to eight successive input signals. Preferably, the excitation signal sequence includes five successive input signals, and voltage values of the five successive input signals are respectively 0.3 V, 0.8 V, 1.2 V, 1.7 V, and 2.2 V.

Optionally, output currents of the output current acquisition points of the first excitation signal, the second excitation signal, and the excitation signal sequence are acquired at intervals of 0.05s.

### Embodiment 2

Based on Embodiment 1, the main difference lies in that, the method further includes a step of applying an alternating-current voltage to the second working electrode before the first excitation signal is input to the working electrode, to measure an impedance value of blood glucose; and obtaining the concentration of the blood glucose based on the output current group and the impedance value. $f = K + {\sum }_{n = 1}^{N} \frac{{X}_{n} {i}_{n}}{{i}_{average}} + {i}_{difference} ⋅ {\sum }_{j = 1}^{J} \frac{{X}_{j} {i}_{j}}{{i}_{average}} + R ⋅ {\sum }_{m = 1}^{M} \frac{{X}_{m} {i}_{m}}{{i}_{average}}$

R is an impedance value, Ω; im is an output current nA in the output current group; a value of M ranges from 10 to 53; and a value of Xm ranges from -120 to 120.

Compared with Embodiment 1, mutual impact between the impedance value and the output current is further considered in Embodiment 2.

### Embodiment 3

Based on Embodiment 2, the method further includes a step of obtaining an actually measured ambient temperature, and obtaining the blood glucose concentration based on the output current group, the impedance value, and the temperature. $f = K + {\sum }_{n = 1}^{N} \frac{{X}_{n} {i}_{n}}{{i}_{average}} + {i}_{difference} ⋅ {\sum }_{j = 1}^{J} \frac{{X}_{j} {i}_{j}}{{i}_{average}} + R ⋅ {\sum }_{m = 1}^{M} \frac{{X}_{m} {i}_{m}}{{i}_{average}} + T ⋅ {G}_{calculate} ⋅ {i}_{difference} ⋅ {\sum }_{u = 1}^{U} \frac{{X}_{u} {i}_{u}}{{i}_{average}}$

T is an ambient temperature °C. iu is an output current nA in the output current group; a value of U ranges from 10 to 53; and a value of Xu ranges from -7 to 3.

Compared with Embodiment 2, mutual impact between the impedance value, the ambient temperature, and the output current is further considered in Embodiment 3.

### Example

A sensor capable of performing the blood glucose concentration calculation method in Embodiment 1 to Embodiment 3 is used to measure a blood sample to be tested. A calculation module is disposed in the sensor. The calculation module includes a calculation formula. A manner and a process of storing the calculation module are prior art techniques and can be implemented by a person skilled in the art. Details are not described again in this embodiment.

A test time and a potential in the test method are controlled as follows: (1) preposing a test neutral of 1.25s, during which an alternating-current voltage may be applied to the second working electrode for measuring an impedance value of the blood glucose (2) applying a first excitation signal to the first working electrode, inputting a potential difference of 0.3 V to the first working electrode and a corresponding electrode, lasting for 0.5s, and synchronously measuring output currents at an interval of 0.05s; (3) after the first excitation signal ends, setting the first working electrode to an open circuit state, and measuring a first potential attenuated signal of the first working electrode at an interval of 0.005s, lasting for 0.5s; (4) when the first working electrode is continuously in an open circuit state, inputting an excitation signal sequence to the second working electrode and the corresponding electrode, the excitation signal sequence including five excitations, excitation voltages thereof sequentially being 0.3 V, 0.8 V, 1.2 V, 1.7 V, and 2.2 V, duration of each excitation being 0.2s, and synchronously measuring output currents at an interval of 0.05s; (5) setting the second working electrode to an open circuit state; (6) after the first working electrode is continuously in an open circuit state for 2.0s, applying a second excitation signal to the first working electrode at a voltage of 0.3 V for 1.25s, and synchronously measuring output currents at an interval of 0.05s; and (7) after the second excitation signal ends, setting the first working electrode to an open circuit state, and measuring a potential attenuated signal of the corresponding electrode at an interval of 0.005s, lasting for 0.5s. A total time of the foregoing test steps is 1.25s+0.5s+2s+1.25s+0.5s=5.5s.

Referring to FIG. 11, FIG. 11 illustrates a linear relationship diagram between a historical i_{average} and a historical glucose concentration, and a slope S is 22.181. The i_{average} in Embodiment 1 to Embodiment 3 is an average value of output currents of last five current acquisition points in the second excitation signal.

In Embodiment 1, f=-18.78+1.068i3/ic+3.46i5/i_{average}-5.58i6/i_{average}-5.14i9/i_{average}+6.51i10/i_{average}-11.95i12/i_{average}-8.05i18/i_{average}+3.355i21/i_{average}+4.292i23/i_{average}-6.792i24/i_{average}+0.991i26/i_{average}+0.3292i30/i_{average}+0.4228i31/i_{average}-2.718i32/i_{average}+1.779i33/i_{average}+0.790i37/i_{average}+18.22i52/i_{average}-0.1262 i_{difference} i2/i_{average}-0.2950 i_{difference} i4/i_{average}+2.740 i_{difference} i8/i_{average}-2.310 i_{difference} i9/i_{average}+1.201 i_{difference} i11/i_{average}+1.653 i_{difference} 118/i_{average}-0.909 i_{difference} 122/i_{average}-1.059 i_{difference} i23/i_{average}+1.466 i_{difference} i24/i_{average}-0.01481 i_{difference} i27/i_{average}-0.04952 i_{difference} i29/i_{average}+0.3606 i_{difference} i32/i_{average}-0.3276 i_{difference} i34/i_{average}-0.298 i_{difference} i39/i_{average}+0.228 i_{difference} i48/i_{average}.

In Embodiment 2, =27.81-33.79i12/ic+11.87i19/i_{average}+*1.321i23*/i_{average}-6.96126/i_{average}-0.2726i29/i_{average}+0.1939i31/i_{average}+1.046i33/i_{average}+28.46i52/i_{average}-0.0742 i_{difference} i2/i_{average}+0.2468 i_{difference} i8/i_{average}+1.290 i_{difference} i11/i_{average}-0.2111 i_{difference} i22/i_{average}-0.3908 i_{difference} i23/i_{average}+0.6853 i_{difference} i26/i_{average}-0.02858 i_{difference} i30/i_{average}+0.0447 i_{difference} i31/i_{average}+0.3847 i_{difference} i33/i_{average}-0.675 i_{difference} i34/i_{average}+0.599 i_{difference} i37/i_{average}-0.580 i_{difference} i39/i_{average}+0.689Ri5/i_{average}-0.839Ri6/i_{average}+8.36Ri13/i_{average}-2.831Ri19/i_{average}+1.103Ri26/i_{average}-0.02016Ri27/i_{average}+0.1339Ri30/i_{average}-0.3000Ri32/i_{average}.

In Embodiment 3, f=18.27-51.0i11/ic-3.232124/i_{average}+4.20i27/i_{average}-4.77i30/i_{average}+12.12i31/i_{average}-13.90i32/i_{average}-440i51/i_{average}+476i52/i_{average}+2.690 i_{difference} i12/i_{average}+2.24 i_{difference} i14/i_{average}-0.541 i_{difference} i23/i_{average}+0.675 i_{difference} i24/i_{average}+0.0220 i_{difference} i28/i_{average}-0.1763 i_{difference} i29/i_{average}+0.1496 i_{difference} i30/i_{average}+0.0723 i_{difference} i31/i_{average}-0.2146 i_{difference} i34/i_{average}+0.890 i_{difference} i39/i_{average}-0.908 i_{difference} i41/i_{average}+11.34Ri11/i_{average}+0.659Ri23/i_{average}-1.100Ri27/i_{average}+1.258Ri30/i_{average}-3.069Ri31/i_{average}+3.511Ri32/i_{average}+112.4Ri51/i_{average}-116.8Ri52/i_{average}-0.0202TG_{calculate} i_{difference} i2/i_{average}+0.0574TG_{calculate} i_{difference} i3/i_{average}-0.0318TG_{calculate} i_{difference} i4/i_{average}+2.381TG_{calculate} i_{difference} i11/i_{average}-6.085TG_{calculate} i_{difference} i12/i_{average}+0.0665TG_{calculate} i_{difference} i19/i_{average}-0.1297TG_{calculate} i_{difference} i22/i_{average}-0.1133TG_{calculate} i_{difference} i23/i_{average}+0.1401TG_{calculate} i_{difference} i26/i_{average}+0.03416TG_{calculate} i_{difference} i27/i_{average}+0.1098TG_{calculate} i_{difference} i29/i_{average}-0.1481TG_{calculate} i_{difference} i30/i_{average}-0.0645TG_{calculate} i_{difference} i32/i_{average}+0.0833TG_{calculate} i_{difference} i33/i_{average}-0.0434TG_{calculate} i_{difference} i36/i_{average}+0.02523TG_{calculate} i_{difference} i53/i_{average}.

To verify the accuracy of the measurement method in this embodiment, the following verification is performed:
1. Determine a blood glucose concentration reference value. The blood glucose concentration reference value is determined by using a YSI2300 blood glucose analyzer.
2. Calculate a deviation. The deviation is an absolute deviation, and deviation=measured value of the blood glucose concentration-blood glucose concentration reference value.

Referring to Table 1, Table 2 illustrates ratios of deviations between Embodiment 1 to Embodiment 3 and blood glucose concentration reference values in various ranges.

**Table 2**

| | Absolute deviation % | Ratio /% |
|---|---|---|
| Embodiment 1 | ≤10 | 66.4% |
| | ≤15 | 87.2% |
| | ≤20 | 95.3% |
| Embodiment 2 | ≤10 | 69.8% |
| | ≤15 | 90.7% |
| | ≤20 | 97.9% |
| | ≤10 | 80.5% |
| Embodiment 3 | ≤15 | 100% |
| | ≤20 | 100% |

Referring to Table 2, it can be seen that, compared with the blood glucose concentration reference value measured by a YSI2300 blood glucose analyzer, the deviation of the blood glucose concentration calculated by the present invention reaches 66% within ±10%, a deviation of the blood glucose concentration calculated by the present invention reaches 96% within ±15%, and a deviation of the blood glucose concentration calculated by the present invention reaches 97% within ±20%.

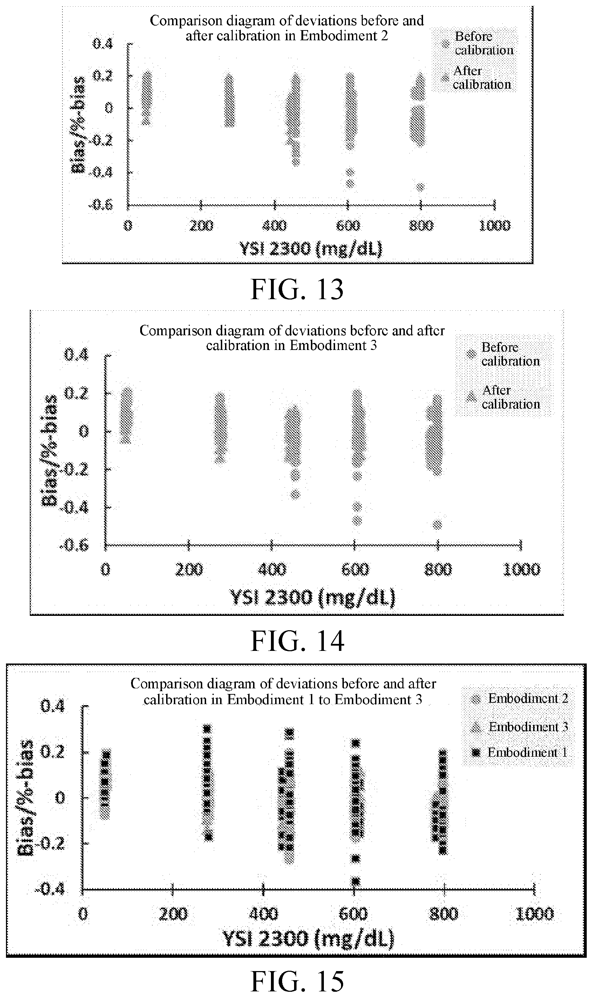

FIG. 12 to FIG. 14 are respectively diagrams of deviations obtained before and after calibration in Embodiment 1 to Embodiment 3. Before calibration, the figures illustrate a diagram of a deviation comparison between a calculated value G and a reference value of a blood glucose concentration, and after calibration, the figures illustrate a diagram of a deviation comparison between a measured value G of the blood glucose concentration and the blood glucose concentration reference value in the present invention. FIG. 15 is a diagram of deviation comparison before and after the calibration in Embodiment 1 to Embodiment 3. It can be seen that, based on the calibrated blood glucose concentration calculation method of the present invention, mutual impact relationships among a plurality of interfering factors correlated with the blood glucose concentration are comprehensively considered, thereby further improving the accuracy and the precision of the blood glucose concentration in a tested biological sample.

### Embodiment 4

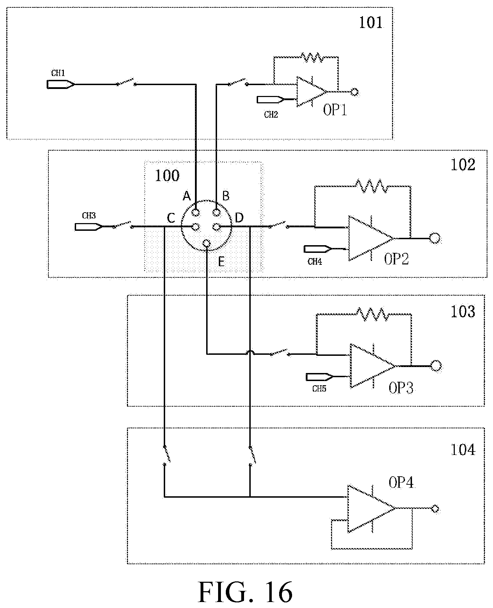

As shown in FIG. 16, an embodiment of this application provides a measurement circuit for a blood glucose test apparatus. The measurement circuit is built in the blood glucose test apparatus. The blood glucose test apparatus includes a test paper response unit 100 and a processing unit. The test paper response unit 100 is configured to be connected to blood glucose test paper. The blood glucose test paper and the measurement circuit may have a detachable structure independent of each other, or may have an integrated and fixed structure.

As shown in FIG. 1, the blood glucose test paper includes a plurality of electrodes, respectively an electrode A to an electrode E. Upper ends of the plurality of electrodes are configured to place a blood glucose sample to be measured, and lower ends thereof are connected to corresponding electrode interfaces (respectively, an interface A to an interface E) in the test paper response unit 100. An electrode in the blood glucose test paper is in a one-to-one mapping with an electrode interface in the test paper response unit 100. For ease of description, during description below, the blood glucose test paper has been connected to the test paper response unit 100 by default if no additional description is provided.

Based on this, a plurality of electrodes included in the test paper response unit 100 are combined. Based on different combined electrodes, a corresponding electrode pair may be formed. Based on different structures of processing units connected to each electrode pair, the processing units also have corresponding functions.

Specifically, the test paper response unit includes at least an impedance test electrode pair and a current test electrode pair. The impedance test electrode pair is composed of a second working electrode A and a second counter electrode B, which are connected to the impedance measurement module 101 in the processing unit. The current test electrode is composed of a first working electrode C and a first counter electrode D, which are connected to a current measurement module 102 and a potential tracking module 104 in the processing unit.

The impedance measurement module 101 is configured to test an impedance of a blood glucose sample, and as shown in FIG. 16, including a signal generator. Based on requirements, the signal generator can apply an alternating-current signal or a direct-current signal to the impedance test electrode pair.

The current measurement module 102 can apply an excitation voltage to the current test electrode pair for testing a current of a blood glucose sample. After the excitation voltage is disconnected, the potential tracking module 104 can test an attenuation potential of the current test electrode pair.

Compared with a conventional solution in which a blood glucose value in a blood glucose sample is obtained only by measuring a current, an attenuation potential of a current test electrode pair may alternately be measured. The attenuation potential may be used as one of the blood glucose calibration parameters, and can improve the blood glucose measurement accuracy.

In an embodiment, the impedance test electrode pair includes a second working electrode (that is, an electrode A shown in FIG. 16) and a second counter electrode (that is, an electrode B shown in FIG. 16). In this case, the impedance measurement module 101 further includes a first operational amplifier OP1, a signal generator is connected to the second working electrode, and the first operational amplifier OP1 is connected to the second counter electrode. The signal generator applies an alternating-current signal or a direct-current signal to two ends of the impedance test electrode pair based on requirements, and implements impedance measurement by using the operational amplifier OP1.

In an embodiment, the current test electrode pair includes a first counter electrode (that is, an electrode C shown in FIG. 16) and a first working electrode (that is, an electrode D shown in FIG. 16). The current measurement module 102 includes a second operational amplifier OP2, the first counter electrode is connected to ground through a switch, and the second operational amplifier OP2 is connected to the first working electrode through a switch. After the blood in a blood glucose sample is drawn into the test paper response unit, an excitation voltage V is applied between current test electrode pairs, and a reduction reaction occurs between gluconase and the glucose in the blood glucose sample, to generate a weak current. The weak current in the current test electrode pair is measured by using the second operational amplifier OP2, and the weak current is converted into a blood glucose value.

Further, the potential tracking module 104 includes a fourth operational amplifier OP4. The potential tracking module 104 is separately connected to the first working electrode and the first counter electrode through switches. By using a double-pulse excitation manner, after the excitation voltage at two ends of the current test electrode pair is disconnected, the attenuation potential of the first working electrode and the first counter electrode can be measured by using the potential tracking module 104, so that the attenuation potential is used for blood glucose value measurement, thereby increasing the measurement accuracy.

In an embodiment, the test paper response unit 100 further includes a sample feeding measurement electrode (that is, an electrode E shown in FIG. 1 and FIG. 16). The processing unit further includes a sample feeding measurement module 103. The sample feeding measurement module 103 includes a third operational amplifier OP3.

The sample feeding measurement module 103 is connected to the sample feeding measurement electrode through a switch. The sample feeding measurement module 103 measures, based on the third operational amplifier OP3 and by using the sample feeding measurement electrode, whether a blood glucose sample covers the test paper response unit 100, and further determines whether blood in the test paper response unit is full. Measurement of a corresponding blood glucose value is performed only after the blood in the test paper response unit is full.

In an embodiment, one of the impedance test electrode pairs is electrically connected to one of the current test electrode pairs. As shown in FIG. 1, the electrode A is electrically connected to the electrode C, and the electrode B is electrically connected to the electrode D.

The foregoing descriptions are merely embodiments of this application, and the protection scope of this application is not limited to these specific embodiments, but is determined according to the claims of this application. For a person skilled in the art, various modifications and variations can be made to this application. Any modification, equivalent replacement, improvement and the like made within the technical idea and principle of this application shall fall within the scope of protection of this application.

## Claims

1. A method for testing an analyte in a biological sample, comprising the following steps:
contacting working electrodes with the biological sample, wherein the working electrodes comprise a first working electrode and a second working electrode, the first working electrode is covered with a first chemical agent capable of reacting with an analyte, and the second working electrode is a blank electrode and is covered with an inactive material that does not chemically react with the analyte or covered with a second chemical agent that is different from the first chemical agent, and inputting at least two excitation signals to the first working electrode, wherein after each excitation signal is completed, the first working electrode is in an open circuit state;
inputting, between the at least two excitation signals, an excitation signal sequence to the second working electrode, wherein after the excitation signal sequence is completed, the second working electrode is in an open circuit state; and the excitation signal sequence comprises at least two successive or non-successive input signals, each input signal being a constant value;
measuring at least one output signal in response to the excitation signals, to obtain an output signal group and at least one output signal sequence in response to the excitation signal sequence; and
obtaining a concentration of the analyte in the biological sample based on the output signal group and the output signal sequence.

2. The method according to claim 1, further comprising: measuring attenuated signals after the at least two excitation signals are completed, to obtain an attenuated signal group, and
obtaining the concentration of the analyte in the biological sample based on the output signal group, the attenuated signal group, and the output sequence signal,
preferably, after the excitation signal is completed, the attenuated signals are measured within not more than one second after the first working electrode is turned off.

3. The method according to claim 1, wherein the at least two excitation signals comprise a first excitation signal and a second excitation signal, and at least one first output signal in response to the first excitation signal and at least one second output signal in response to the second excitation signal are measured, and
the concentration of the analyte in the biological sample is obtained based on the first output signal, the second output signal, and the output signal sequence.

4. The method according to claim 3, further comprising measuring a first attenuated signal after the first excitation signal is completed and a second attenuated signal after the second excitation signal is completed, and obtaining the concentration of the analyte in the biological sample based on the first output signal, the second output signal, the first attenuated signal, the second attenuated signal, and the output signal sequence.

5. The method according to claim 4, further comprising: measuring a third attenuated signal after the excitation signal sequence is completed; obtaining the concentration of the analyte in the biological sample based on the first output signal, the second output signal, the first attenuated signal, the second attenuated signal, the third attenuated signal, and the output signal sequence, preferably, after the excitation signal sequence is completed, the third attenuated signal is measured within not more than one second after the second working electrode is turned off.

6. The method according to claim 3, wherein a voltage value of the first excitation signal is equal to that of the second excitation signal, and an application time of the first excitation signal is less than an application time of the second excitation signal,
preferably, the voltage value of the first excitation signal is 0.2 V to 0.4 V, and the application time is at least 0.1s to 1s; the voltage value of the second excitation signal is 0.2 V to 0.4 V, and the application time is 0.1s to 2s; and
further preferably, the voltage values of the first excitation signal and the second excitation signal are both 0.3 V, the application time of the first excitation signal is 0.5s, and the application time of the second excitation signal is 1.25s.

7. The method according to claim 1, wherein the excitation signal sequence comprises at least two successively applied input signals, output voltages of the excitation signal sequence successively increase or successively decrease, and the voltage ranges from 0.1 V to 3 V,
preferably, the excitation signal sequence comprises four to eight successive input signals; and
further preferably, the excitation signal sequence comprises five successive input signals, and voltage values of the five successive input signals are respectively 0.3 V, 0.8 V, 1.2 V, 1.7 V, and 2.2 V.

8. The method according to claim 1, wherein the excitation signal sequence comprises at least two non-successively applied input signals, output voltages of the excitation signal sequence non-successively increase or non-successively decrease, and the voltage ranges from 0.1 V to 3 V,
preferably, the excitation signal sequence comprises four to eight successive input signals; and
further preferably, the excitation signal sequence comprises eight successive input signals, and voltage values of the six successive input signals are respectively 0.3 V, 0.7 V, 1.1 V, 0.9 V, 1.2 V, 1.5 V, 1.9 V, and 2.3 V.

9. The method according to claim 1, wherein the excitation signal sequence comprises four to six non-successive input signals,
preferably, the excitation signal sequence comprises five non-successive input signals, and voltage values of the five non-successive input signals are respectively 0.3 V, 0.8 V, 1.2 V, 1.7 V, and 2.2 V.

10. The method according to claim 1, wherein a total application time of the excitation signal sequence is at least 1s, preferably an application time of each input signal in the excitation signal sequence is 0.1s to 0.3s; and
further preferably, the application times of all of the input signals in the excitation signal sequence are equal, each being 0.2s.

11. The method according to claim 1, wherein before the inputting at least two excitation signals to the first working electrode, the method further comprises a step of applying an alternating-current voltage to the second working electrode, to measure an impedance value of the biological sample; and
obtaining the concentration of the analyte in the biological sample based on the output signal group, the output signal sequence, and the impedance value.

12. The method according to claim 1, wherein the first chemical agent comprises an oxidation enzyme and a mediator, the oxidation enzyme is selected from at least one of a FAD dehydrogenase and a PQQ dehydrogenase, and the mediator is selected from one of hexaammineruthenium (III) chloride, potassium ferricyanide, a ferrocene derivative, phenazine methosulphate, and a polyethersulfone resin.

13. The method according to claim 1, wherein the inactive material is selected from at least one of carboxymethyl cellulose, hydroxymethyl cellulose, polyethylene glycol, and polyvinyl pyrrolidone.

14. A sensor for testing an analyte in a biological sample, comprising:
a biosensor, the biosensor comprising a first working electrode, wherein the first working electrode is covered with a first chemical agent capable of reacting with an analyte, the first working electrode is capable of receiving at least two excitation signals, and after each excitation signal is completed, the first working electrode is in an open circuit state; and
an electro-chemical sensor, the electro-chemical sensor comprising a second working electrode, wherein the second working electrode is a blank electrode and is covered with an inactive material that does not chemically react with the analyte or covered with a second chemical agent that is different from a first chemical agent, and between the at least two excitation signals, the second working electrode is capable of receiving an excitation signal sequence, the excitation signal sequence comprising at least two input signals, and each of the input signals being a constant value, and measuring at least one output signal in response to the excitation signals, to obtain an output signal group and at least one output signal sequence in response to the excitation signal sequence, to determine a concentration of a target analyte in the biological sample.

15. The sensor according to claim 14, wherein after receiving each excitation signal, the first working electrode measures an attenuated signal of each excitation signal, to obtain an attenuated signal group, and determines the concentration of the analyte in the biological sample based on the output signal group, the attenuated signal group, and the output sequence signal.

16. A system for testing an analyte in a biological sample, comprising:
a biosensor, the biosensor comprising a first working electrode and a first counter electrode corresponding to the first working electrode, and the first working electrode being covered with a first chemical agent capable of reacting with a target analyte;
an electro-chemical sensor, the electro-chemical sensor comprising a second working electrode and a second counter electrode corresponding to the second working electrode, and the second working electrode being a blank electrode or covered with an inactive material that does not chemically react with the analyte; and
a signal collection apparatus, the signal collection apparatus being capable of inputting at least two excitation signals to the first working electrode, and between the at least two excitation signals, being capable of inputting an excitation signal sequence to the second working electrode, the excitation signal sequence comprising at least two input signals, each input signal being a constant value.

17. The system according to claim 16, wherein the signal collection apparatus comprises: an operational amplifier, a signal generation module, a data acquisition module, and a data storage module.
